# EUROPEAN PATENT APPLICATION

(11) **EP 1 917 969 A1**
(43) Date of publication of application: **07.05.2008**
(21) Application number: 07118509.4
(22) Date of filing: 15.10.2007
(51) Int. Cl.: A61K 35/74, A61K 45/06, A61P 1/12

(54) **Compositions for preventing depletion of the intestinal flora and organ damage following antibiotic treatment**

(30) Priority: 23.10.2006 IT MI20602031
(71) Applicant: Velleja Research SRL, 20122 Milano (IT)
(72) Inventor: Di Pierro, Francesco, 20090, TREZZANO SUL NAVIGLIO (MI) (IT)
(74) Representative: Minoja, Fabrizio

(57) **Abstract**

The present invention relates relates to compositions containing probiotic strains, prebiotic substances, vitamin complexes with trace elements, lycopene, glutathione and possibly bioflavonoids, which are designed to prevent depletion of the intestinal flora and organ damage following antibiotic treatment.

## Description

### FIELD OF INVENTION

The present invention relates to compositions containing probiotic strains, prebiotic substances, vitamin complexes with trace elements, lycopene, glutathione and possibly bioflavonoids, which are designed to prevent depletion of the intestinal flora and organ damage following antibiotic treatment.

### INTRODUCTION

The exceptional importance of intestinal commensal flora to health, especially in immunological terms, has long been known. The gastroenteric commensal flora of a healthy adult consists of at least 10¹⁸ bacteria, 99% of which belong to some 30-40 species. This flora comprises anaerobic germs (bifidobacteria, clostridia, bacterioids, eubacteria and Gram-positive cocci) and aerobic germs (lactobacilli, streptococci, staphylococci and coliforms), distributed to an increasing extent along the gastroenteric axis, until the highest content is reached at the colon (Bacteroides spp., Clostridium spp., Fusobacterium spp., Klebsiella spp., Staphylococci, yeasts and Escherichia coli). This microbial flora can be conveniently divided into two categories: "resident" flora, which is almost invariably present and, if altered, can be promptly restored, and "transient" flora, which can colonise the host for short periods, but tends to be eliminated by competition from the resident micro-organisms or the host's defence mechanisms.

The exact composition of the intestinal flora is influenced by numerous factors. One important factor is the ability of bacteria to adhere to the epithelial cells. Some bacteria present marked tropism (affinity) for particular epithelial cells: the normal flora can then interfere with the potentially pathogenic micro-organisms by competing with them for the receptors on the cell surface. Commensal flora can also interfere with pathogenic micro-organisms by producing bacteriocins, substances which inhibit the growth of other bacteria (usually of the same species), or by competing for the same nutrients. Due to these mechanisms, the normal vaginal flora represents an effective barrier that reduces the probability of colonisation of the host surfaces by pathogenic micro-organisms ("colonisation-resistance").

As will be obvious, any phenomenon that reduces the effect of these microbial factors on the gastroenteric ecosystem can lead to serious problems for the health of the individual.

For example, treatment with broad-spectrum antibiotics eliminates all the commensal bacteria of the gastroenteric flora which are sensitive to the antimicrobial agent used. In this case, colonisation resistance is reduced, and potentially lethal micro-organisms are free to colonise the mucosa. When the treatment is discontinued, the resident flora can be restored, with time. Unfortunately, however, aerobic Gram-negative bacteria grow faster and colonise the mucous membranes sooner than anaerobic Gram-negative bacteria, which proliferate more slowly, although they constitute 99% of the commensal flora. In patients whose immune defences are even only partly impaired, this imbalance can cause Gram-negative bacteraemia. Other possible consequences associated with suppression of the normal flora by broad-spectrum antibiotics include excessive growth of yeasts with the appearance of mycosis, or excessive growth of the anaerobic Gram-negative bacterium Clostridium difficile, which is unfortunately relatively antibioticresistant. Its presence can lead to a series of very common disorders, ranging from diarrhoea to colitis.

The conventional solution to problems of depletion of the intestinal flora and possible organ damage associated with antibiotic treatment is to administer various species of milk enzymes (Lactobacilli). However, these bacteria proliferate more slowly than pathogenic bacteria, which become established more quickly; there is consequently a need for new preparations which restore the bacterial flora rapidly and efficiently.

Prebiotics are substances used to provide suitable selective nutrition to probiotic bacteria in order to support their resistance, colonising capacity and reproductive capacity in the intestine. In chemical terms, prebiotic substances correspond to indigestible dietary fibres: after being swallowed, these substances pass through nearly all of the gastrointestinal apparatus intact, without undergoing any digestive process. When they reach the colon, they constitute the main nutrient substrate of the bacteria whose presence is to be supported, which can use these substances and cleave them into simple sugars, so that they can be used as a nutrient substrate.

The most widely studied prebiotics are galacto-oligosaccharides (GOS) and fructo-oligosaccharides (FOS). The inclusion of GOS guarantees the presence of the nutritional substrate essential to the physiological balance of the entire microbial flora. When GOS, which are non-hydrolysable polysaccharides, break down (which can only result from bacterial action), they reduce the intestinal pH, thus combating excessive basicity, which is often a source of pathogen growth. FOS are also widely used prebiotics. In chemical terms they are short-chain fructans, and consequently soluble, with a degree of polymerisation not exceeding 5 carbohydrate units. The addition of prebiotics considerably modifies the composition of the intestinal microflora, leading to a great increase in the intestinal probiotic flora.

Bioflavonoids, especially the proanthocyanidins extracted from Vitis vinifera, are hydrophilic compounds with a potent antioxidant action. Lycopene, extracted from the skin of the tomato (Lycopersicum esculentum), is a very potent lyophilic antioxidant. When associated in suitable proportions, these two compounds are highly effective as an anti-oxidant in combating oxidative stress of any origin.

### DESCRIPTION OF THE INVENTION

It has now been found that compositions containing:
a) probiotic strains;
b) prebiotic substances,
c) vitamin complexes with trace elements;
d) lycopene;
e) glutathione;
   and possibly
f) bioflavonoids
are highly effective in preventing depletion of the intestinal flora and organ damage following antibiotic treatment. This invention therefore relates to compositions designed to prevent depletion of the intestinal flora and organ damage following antibiotic treatment.

According to a preferred aspect of this invention, the probiotic strains will be selected from the genera Lactobacilli, Saccharomycetes and yeasts, Streptococcus thermophilus, Enterococcus faecium and Bifidobacterium bifidum. According to a particularly preferred aspect, the Lactobacilli will be selected from Lactobacillus acidophilus, bulgaricus, casei, rhamnosus, sporogenes and reuteri.

According to a preferred aspect of the invention, the prebiotic substances will be indigestible dietary fibres chosen from galacto-oligosaccharides (GOS) and fructo-oligosaccharides (FOS).

According to a preferred aspect of the invention, the vitamin complexes will be chosen from the vitamins in Group B, while the trace elements will be chosen from Cu, Zn and Se.

According to a preferred aspect thereof, the compositions according to the invention will also contain bioflavonoids, especially proanthocyanidins. According to a particularly preferred aspect, the proanthocyanidins will be those extracted from Vitis vinifera, in particular proanthocyanidin A2. Alternatively, the compositions according to the invention could contain other antioxidant polyphenols such as resveratrol, epigallocatechin gallate and the like.

This invention therefore relates to compositions designed to prevent depletion of the intestinal flora and organ damage following antibiotic treatment.

The effect of the compositions according to the invention is greater than the sum of the effects obtained following separate administration of the individual components of the combination, apparently due to synergy between the various components.

The compositions according to the invention will preferably contain the various components in the following ranges:
a) probiotic strains: 1 to 1000 mg (150 billion CFU/g);
b) prebiotic substances: 0.1 g to 10 g;
c) vitamin complexes with trace elements; 1% to 300% of the RDA (Recommended Daily/Dietary Allowance);
d) lycopene: 0.1 to 100 mg;
e) glutathione: 0.1 to 500 mg;
   and possibly
f) bioflavonoids: 0.1 to 1000 mg.

According to a particularly preferred aspect, the compositions according to the invention will contain the following constituents in the following ranges:
a) lactobacilli: 10 to 100 mg;
b) GOS and/or FOS: 1 to 4 g;
c) vitamin B complex with Cu and/or Zn and/or Se: 100 to 150% RDA;
d) lycopene: 1 to 10 mg;
e) glutathione: 10 to 30 mg;
   and possibly
f) leucoanthocyanidin: 50 to 150 mg.

The compositions according to the invention can be formulated suitably for oral administration, and will be prepared according to conventional methods well known in pharmaceutical technology, such as those described in Remington's Pharmaceutical Handbook, Mack Publishing Co., N.Y., USA, using excipients, diluents, fillers and anti-caking agents acceptable for their final use.

The compositions according to the invention can be formulated, for example, in the form of tablets, capsules, granulates or sachets (possibly separate, to be taken simultaneously or separately, each containing different constituents).

Some examples of formulations according to the invention are set out below.

### Example 1

| PRODUCT: PROBIOTIC Type of formulation: 5.5 g sachets | | |
|---|---|---|
| | | |
| NAME OF THE COMPONENT | mg/bact | % |
| | | |
| Lactobacillus acidophilus | 100.000 | 1.818 |
| Inulin | 2000.000 | 36.364 |
| Vitamin B1 | 1.400 | 0.025 |
| Vitamin B2 | 1.600 | 0.029 |
| Vitamin B6 | 2.000 | 0.036 |
| Vitamin B12 | 0.0001 | 0.000 |
| Folic acid | 0.150 | 0.003 |
| Vitamin C | 60.000 | 1.091 |
| Vitamin E | 10.000 | 0.182 |
| Sodium selenite | 0.120 | 0.002 |
| Zinc gluconate | 125.000 | 2.273 |
| Magnesium oxide | 497.347 | 9.043 |
| Manganese gluconate | 25.5320 | 0.464 |
| Copper citrate | 3.401 | 0.062 |
| Lycopene | 6.000 | 0.109 |
| Glutathione | 30.000 | 0.545 |
| Vine proanthocyanidins | 100.000 | 1.818 |
| Saccharose | 1802.450 | 32.772 |
| Maltodextrins | 500.000 | 9.091 |
| Citric acid | 50.000 | 0.909 |
| Silicon dioxide | 20.000 | 0.364 |
| Flavouring | 150.000 | 2.727 |
| Acesulfame K | 15.000 | 0.273 |
| TOTAL | 5500.000 | |

### Example 2

| PRODUCT: PROBIOTIC Type of formulation: 2 g sachet A | | |
|---|---|---|
| | | |
| NAME OF THE COMPONENT | mg/bact | % |
| Lactobacillus acidophilus | 100.000 | 5.000 |
| Inulin | 1300.000 | 65.000 |
| Vitamin B1 | 1.400 | 0.070 |
| Vitamin B2 | 1.600 | 0.080 |
| Vitamin B6 | 2.000 | 0.100 |
| Vitamin B12 | 0.0001 | 0.000 |
| Folic acid | 0.150 | 0.008 |
| Vitamin E | 10.000 | 0.500 |
| Maltodextrins | 564.850 | 28.242 |
| Silicon dioxide | 20.000 | 1.000 |
| TOTAL | 2000.000 | |

### Example 3

| PRODUCT: PROBIOTIC Type of formulation: 5 g sachet B | | |
|---|---|---|
| | | |
| NAME OF THE COMPONENT | mg/bact | % |
| Inulin | 700.000 | 20.000 |
| Vitamin C | 60.000 | 1.714 |
| Sodium selenite | 0.120 | 0.003 |
| Zinc gluconate | 125.000 | 3.571 |
| Magnesium oxide | 497.347 | 14.210 |
| Manganese gluconate | 25.5320 | 0.729 |
| Copper citrate | 3.401 | 0.097 |
| Lycopene | 6.000 | 0.171 |
| Glutathione | 30.000 | 0.857 |
| Vine proanthocyanidins | 100.000 | 2.857 |
| Saccharose | 1662.600 | 47.503 |
| Citric acid | 50.000 | 1.429 |
| Silicon dioxide | 20.000 | 0.571 |
| Flavouring | 200.000 | 5.714 |
| Acesulfame K | 20.000 | 0.571 |
| TOTAL | 3500.000 | |

### Example 4

| PRODUCT: PROBIOTIC Type of formulation: 610 g capsules | | |
|---|---|---|
| | | |
| NAME OF THE COMPONENT | mg/bact | % |
| | | |
| Lactobacillus acidophilus | 50.000 | 8.197 |
| Vitamin B1 | 0.700 | 0.115 |
| Vitamin B2 | 0.800 | 0.131 |
| Vitamin B6 | 1.000 | 0.164 |
| Vitamin B12 | 0.0001 | 0.000 |
| Folic acid | 0.075 | 0.012 |
| Vitamin C | 30.000 | 4.918 |
| Vitamin E | 5.000 | 0.820 |
| Sodium selenite | 0.060 | 0.010 |
| Zinc gluconate | 62.500 | 10.246 |
| Magnesium oxide | 248.674 | 40.766 |
| Manganese gluconate | 12.7660 | 2.093 |
| Copper citrate | 1.701 | 0.279 |
| Lycopene | 3.000 | 0.492 |
| Glutathione | 15.000 | 2.459 |
| Vine proanthocyanidins | 50.000 | 8.197 |
| Microcrystalline cellulose | 66.725 | 10.939 |
| Dicalcium phosphate | 50.000 | 8.197 |
| Silicon dioxide | 6.000 | 0.984 |
| Magnesium stearate | 6.000 | 0.984 |
| Gelatin shell | 110 | |
| TOTAL | 610.000 | |

## Claims

1. Compositions containing:
a) probiotic strains;
b) prebiotic substances,
c) vitamin complexes with trace elements;
d) lycopene;
e) glutathione;
and possibly
f) bioflavonoids;
to prevent depletion of the intestinal flora and organ damage following antibiotic treatment.

2. Compositions as claimed in claim 1, wherein the various components are present in the following quantity ranges:
a) probiotic strains; 1 to 1000 mg (150 billion CFU/g);
b) prebiotic substances: 0.1 g to 10 g;
c) vitamin complexes with trace elements; 1 to 300% of RDA;
d) lycopene: 0.1 to 100 mg;
e) glutathione: 0.1 to 500 mg;
and possibly
f) bioflavonoids: 0.1 to 1000 mg.

3. Compositions as claimed in claims 1 and 2, wherein the probiotic strains are selected from the genera Lactobacilli, Saccharomycetes and yeasts, Streptococcus thermophilus, Enterococcus faecium and Bifidobacterium bifidum.

4. Compositions as claimed in claim 3, wherein the Lactobacilli are selected from Lactobacillus acidophilus, bulgaricus, casei, rhamnosus, sporogenes and reuteri.

5. Compositions as claimed in claims 1 and 2, wherein the prebiotic substances are chosen from galacto-oligosaccharides (GOS) and fructo-oligosaccharides (FOS).

6. Compositions as claimed in claims 1 and 2, wherein the vitamin complexes are chosen from the vitamins in Group B, and the trace elements are chosen from Cu, Zn and Se.

7. Compositions as claimed in claims 1 and 2, wherein the bioflavonoids are proanthocyanidins.

8. Compositions as claimed in claim 7, wherein the proanthocyanidins are present as proanthocyanidin A2 extracted from Vitis vinifera.

9. Compositions as claimed in claims 1-8, containing the following components in the following quantity ranges:
a) lactobacilli: 10 to 100 mg;
b) GOS and/or FOS: 1 to 4 g;
c) vitamin B complex with Cu and/or Zn and/or Se: 100 to 150% RDA;
d) lycopene: 1 to 10 mg;
e) glutathione: 10 to 30 mg;
and possibly
f) leucoanthocyanidin: 50 to 150 mg.

10. Use of:
a) probiotic strains;
b) prebiotic substances,
c) vitamin complexes with trace elements;
d) lycopene;
e) glutathione;
and possibly
f) bioflavonoids; to prepare a medicinal product designed to prevent depletion of the intestinal flora and organ damage following antibiotic treatment.
